Europäisches Patentamt

European Patent Office

Office européen des brevets

(11) Publication number: **0 122 344
B1**

(12) **EUROPEAN PATENT SPECIFICATION**

(45) Date of publication of patent specification: 01.07.87

(21) Application number: **83302151.2**

(22) Date of filing: **15.04.83**

(51) Int. Cl.⁴: **A 61 L 15/06, C 09 J 3/18,
C 09 J 3/14, C 09 J 3/12,
C 09 J 3/02, C 08 L 35/08,
C 08 L 23/22, C 08 L 89/00**

(54) **Adhesive composition.**

(43) Date of publication of application:
**24.10.84 Bulletin 84/43**

(45) Publication of the grant of the patent:
**01.07.87 Bulletin 87/27**

(84) Designated Contracting States:
**AT BE CH DE FR GB IT LI LU NL SE**

(56) References cited:
**EP-A-0 017 401
EP-A-0 018 093
FR-A-2 392 076
FR-A-2 393 566**

(73) Proprietor: **E.R. Squibb & Sons, Inc.
Lawrenceville-Princeton Road
Princeton, N.J. 08540 (US)**

(72) Inventor: **Pawelchak, John Michael
J20 Avon Drive
East Windsor New Jersey (US)**
Inventor: **Chen, James Ling
30 Fairview Avenue
East Brunswick New Jersey (US)**
Inventor: **Cryan, John Gerard
43 Farms Road Circle
East Brunswick New Jersey (US)**
Inventor: **LaVia, Anthony Lawrence
66 Farms Road Circle
East Brunswick New Jersey (US)**

(74) Representative: **Thomas, Roger Tamlyn et al
D. Young & Co. 10 Staple Inn
London WC1V 7RD (GB)**

**EP 0 122 344 B1**

Courier Press, Leamington Spa, England.

## Description

This invention is directed to adhesive compositions particularly such compositions for use in the ostomy care field.

The adhesive compositions are a homogeneous blend of one or more pressure sensitive adhesive materials and one or more natural or synthetic polymers capable of developing elastomeric properties when hydrated such as gluten and long chain polymers of methyl vinyl ether/maleic acid. The composition may also include one or more water soluble hydrocolloid gums and may additionally contain one or more water swellable cohesive strengthening agents. Additionally, one or more thermoplastic elastomers may be included with the pressure sensitive adhesive materials.

More specifically, the invention provides an adhesive composition suitable for medical purposes, comprising a substantially homogeneous mixture on a percent weight basis of from 30% to 70% of a pressure-sensitive viscous adhesive material and, optionally, a thermoplastic elastomer, said pressure-sensitive adhesive material being selected from natural rubber, silicone rubber, acrylonitrile rubber, polyurethane rubber, and polyisobutylenes and said optional thermoplastic elastomer being selected from medium molecular weight polyisobutylene, butyl rubber, and styrene copolymers; and from 3% to 60% by weight of one or more natural or synthetic polymers capable of developing elastomeric properties when hydrated and selected from gluten and long chain polymers of methyl vinyl ether/maleic acid.

The invention also extends to a skin barrier comprising an adhesive layer having a thin continuous or discontinuous polymeric film laminated to one surface of the adhesive, said adhesive being as defined above.

Features of the preferred forms of the invention will now be described in more detail. Reference will hereinafter be made to the drawings in which:—

Fig. 1 is a perspective view of a skin barrier incorporating the adhesive composition of this invention; and

Fig. 2 is a front view of a drainable ostomy pouch having an adhesive faceplate of the composition of this invention.

In the EP—A—0 017 401 and EP—A—0 018 093, respectively, adhesive compositions are described which are also based on a pressure sensitive viscous adhesive material and which in addition to other components may contain a methyl vinyl ether/maleic acid or anhydride copolymer. Said copolymer, however, is modified by reaction with a polyhydric alcohol which leads to ester formation.

This invention is directed to adhesive compositions suitable for various medical applications and, in particular, suited for use in the field of ostomy care. The adhesive compositions of this invention are resistant to erosion by moisture and biological fluids which are excreted from a stoma and can leak from a collection appliance. Also, the adhesive compositions of this invention are non-irritating to the human skin.

Thus, a skin barrier prepared from the adhesive compositions of this invention or a drainable ostomy pouch having an adhesive faceplate made from the adhesive compositions of this invention can remain in place around the stoma for from several days to a week or longer. This diminishes irritation to the sensitive skin around a stoma resulting from the frequent removal of adhesive skin barriers and also represents a cost savings for the ostomate.

The adhesive composition of this invention comprises a homogeneous blend of one or more pressure sensitive adhesive materials and one or more natural or synthetic polymers capable of developing elastomeric properties when hydrated such as gluten and long chain polymers of methyl vinyl ether/maleic acid. The composition preferably also includes one or more water soluble hydrocolloid gums and may additionally include one or more water swellable cohesive strengthening agents. Additionally, one or more thermoplastic elastomers may be included with the pressure sensitive adhesive materials.

The pressure sensitive adhesive component of the composition provides dry adhesion and holds the entire composition together. Various natural or synthetic viscous or elastomeric substances such as natural rubber, silicone rubber, acrylonitrile rubber, polyurethane rubber, polyisobutylene, etc., either possessing dry tack by themselves or developing such tack upon the addition of a plasticizer are suitable for this purpose. Low molecular weight polyisobutylenes having a viscosity average molecular weight of from 36,000 to 58,000 (Florey) possessing pressure sensitive adhesive properties are preferred. Such polyisobutylenes are commercially available under the trademark Vistanex from Exxon as grades LM-MS and LM-MH.

Optionally, one or more thermoplastic elastomers can be included in the pressure sensitive adhesive component. These elastomers impart the properties of rubber-like extensibility and both rapid and complete recovery from modular strains to the pressure sensitive adhesive component. Suitable thermoplastic elastomers include medium molecular weight polyisobutylenes having a viscosity average molecular weight of from 1,150,000 to 1,600,000 (Florey), butyl rubber which is a copolymer of isobutylene with a minor amount of isoprene having a viscosity average molecular weight of from 300,000 to 450,000 (Florey), and styrene copolymers such as styrene-butadiene-styrene (S-B-S), styrene-isoprene-styrene (S-I-S), and styrene-ethylene/butylene-styrene (S-EB-S) which are commercially available, for example, from Shell Chemical Co. under the trademark Kraton as Kraton D1100, D1102, Kraton D1107, Kraton 4000, Kraton G1600, and Kraton G4600. Preferred thermoplastic elastomers are butyl rubber having a viscosity average molecular weight of about 425,000 (commercially available as grade 077), polyisobutylene having a viscosity average molecular weight of about 1,200,000 (commercially available under the trademark Vistanex from Exxon as grade L-100), and styrene-isoprene-styrene (S-I-S) copolymers (commercially available from Shell as Kraton D1107).

The pressure sensitive adhesive component including the optional thermoplastic elastomer is present at from 30% to 70% by weight of the composition, preferably from 40% to 50% by weight. The thermoplastic elastomer can be employed at up to three times the weight of the pressure sensitive elastomeric substances but preferably the thermoplastic elastomer if present will be at from 20% to 40% by weight of the pressure sensitive elastomeric substance.

The natural or synthetic polymers which develop elastomeric properties when hydrated are present at from 3% to 60% by weight of the adhesive composition. The preferred materials are the long chain polymers of methyl vinyl ether/maleic acid commercially available under the trademark Gantrez from GAF Inc. The maleic acid moiety in the polymer may be intact (Gantrez S-97), may be an anhydride (Gantrez AN-169), or may be a metal salt such as the mixed sodium/calcium salts (Gantrez AT-955). These materials are hydrophilic and when hydrated form extensible elastic masses with substantial tack to skin and other surfaces.

The adhesive composition may also include up to 50% by weight of one or more water soluble hydrocolloid gums. Suitable hydrocolloid gums include sodium carboxymethylcellulose, pectin, gelatin, guar gum, locust bean gum, and gum karaya. The adhesive composition may also include up to 30% by weight of one or more water swellable cohesive strengthening agents provided that the water soluble hydrocolloid gums and water swellable cohesive strengthening agents together are present at no more than 60% by weight of said adhesive composition. Suitable water swellable cohesive strengthening agents include finely divided substantially water insoluble cross-linked sodium carboxymethylcellulose such as that commercially available under the trademark Aqualon or that described in U.S. Patent 3,589,364 and available commercially from the Buckeye Cellulose Corp., finely divided substantially water insoluble starch-acrylonitrile graft copolymer such as that described in U.S. Patent 3,661,815 and commercially available from the Grain Processing Corp, and finely divided substantially water insoluble cross-linked dextran such as that commercially available under the trademark Sephadex. The water soluble hydrocolloids provide additional wet tack for the adhesive composition and they along with the water swellable cohesive strengthening agents function to control the rate of hydration of the methyl vinyl ether/maleic acid polymeric material.

Preferably, if the adhesive composition contains one or more water soluble hydrocolloid gums then such gums will be present at from 15% to 40% by weight of the composition and the combination of the water soluble hydrocolloid gums and water swellable cohesive strengthening agents will be no more than 55% by weight of the adhesive composition. Also, in these formulas the methyl vinyl ether/maleic acid polymers will be present at from 10% to 35% by weight of the adhesive composition.

Small amounts, i.e., less than 5% by weight of the adhesive composition, of other ingredients may be included in the adhesive composition. For example, a plasticizer such as mineral oil, a tackifying resin such as Piccolyte, an antioxidant such as butylated hydroxyanisole or butylated hydroxytoluene, a deodorant such as chlorophyllins, or a perfume agent may be included. In addition, small amounts of a pharmacologically active ingredient can be included in the adhesive composition. For example, an antibiotic or antimicrobial agent such as neomycin, an antiseptic agent such as povidone iodine, and an antiinflammatory agent such as hydrocortisone or triamcinolone acetonide.

The adhesive compositions are prepared by forming a premix of the gluten or methyl vinyl ether/maleic acid polymeric material, the water soluble hydrocolloid gums, and the cohesive strengthening agents and any other optional substances. The premixed powder is then placed in a heavy duty high shear sigma blade or equivalent type mixer. The viscous pressure sensitive adhesive component is then added in two or three equal segments. Mixing is allowed to proceed for approximately ten minutes between each addition of the viscous material. The resultant dough-like mass is then extruded and rolled or pressed to desired thickness. In working with large batches of material, the dough-like mass may be kneaded prior to the extrusion step. Alternatively, the process may be varied by first working the viscous pressure sensitive adhesive material in the mixer for about ten minutes and then adding the powder premix to two or three equal segments with agitation for about 15 minutes between each addition. When the pressure sensitive adhesive component includes an amount of optional thermoplastic elastomer, such elastomer is first blended by geometric dilution with the pressure sensitive adhesive material in a heated high shear sigma blade or equivalent type mixer.

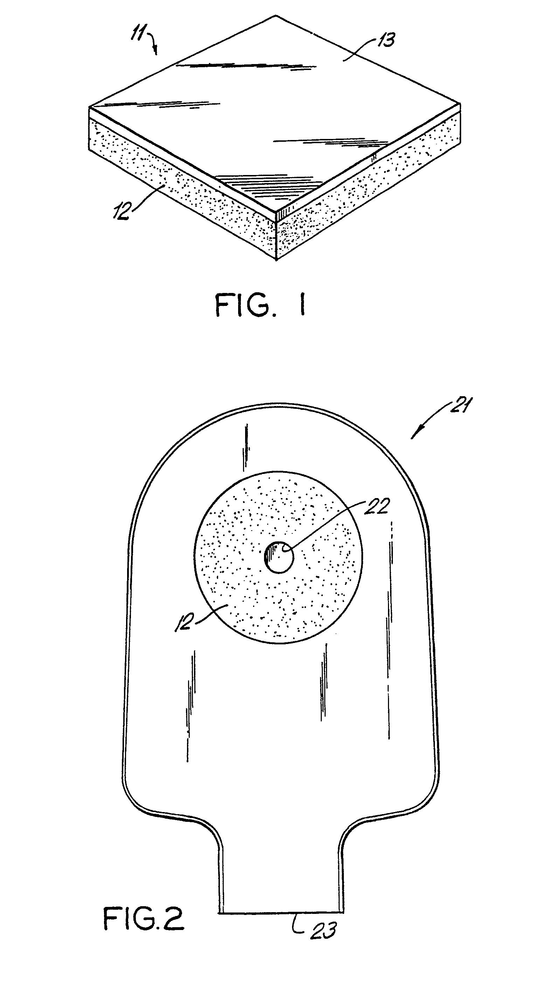

As shown in Figure 1, if desired, a thin continuous or discontinuous film 13 of polymeric material such as polyethylene, polypropylene, polyurethane, polyvinylchloride, etc., can be laminated onto one side of the adhesive composition 12 as taught by Chen in U.S. Patent 3,339,546. In the skin barrier 11, the adhesive layer 12 will vary in thickness from 10 to 120 mils (0.254 to 3.048 mm) and the film 13 will vary in thickness from 1 to 10 mils (0.0254 to 0.254 mm). The exposed side of the adhesive composition or both sides if the film is omitted is covered with a piece of silicone coated release paper.

A flange can be affixed to the film 13 for attachment of an ostomy bag as is known in the art. Preferably, a projecting rim shaped coupling member is affixed to film 13 as described by Steer et al. in British Patent 1,571,657. An ostomy bag having a coacting channel shaped coupling member can then be secured to the skin barrier.

Figure 2 shows the adhesive composition 12 affixed as a mounting faceplate on a drainable ostomy pouch 21. The pouch 21 is formed by conventional procedures such as by heat sealing two films or polymeric material around their periphery except for a bottom opening 23. The mounting faceplate and pouch include an opening 22 which is enlarged by the ostomate so as to fit snuggly around the stoma. The drainable opening 23 is closed by a clip as known in the art. Of course, the adhesive composition 12 may be employed as a faceplate on a disposable ostomy pouch.

While the adhesive compositions of this invention have been particularly described for use in the ostomy field, they are also useful for other medical purposes. For example, the adhesive compositions may be packaged in strip form and employed to hold a male incontinence device in place as described by Rodgers et al. in U.S. Patents 3,835,857 and 3,863,638. The adhesive compositions may also be employed to affix various medical devices to the body such as a female incontinence device as described by Cooney et al. in U.S. Patent 4,198,979, a wound drainage system as described by Harvey in U.S. Patent 3,568,675 and by Nordby in U.S. Patent 3,954,105, a catheter, or an electronic probe. The adhesive compositions of this invention can be used on the mucous membrane in the mouth, in the vagina, or in various surgical procedures such as on the surface of an internal organ such as the liver.

The adhesive compositions of this invention may be sterilized by means of gamma radiation.

The following examples are illustrative of the invention. Other suitable adhesive compositions can be obtained by minor variations in the amounts of ingredients employed.

Example 1

This example was directed to preparing an adhesive mass having the following composition:

| Ingredient | Percent by weight |
|---|---|
| Polyisobutylene (Vistanex LM-MH) | 40 |
| Guar gum | 25 |
| Sodium carboxymethylcellulose | 10 |
| Cross-linked sodium carboxymethylcellulose (Aqualon R) | 15 |
| Sodium, calcium poly (methyl vinyl ether/maleic acid [Gantrez AT-955] | 10 |
| | 100 |

A premix was prepared by blending 2.5 kg. of finely divided guar gum, 1.0 kg. of sodium carboxymethylcellulose, 1.5 kg. of cross-linked sodium carboxymethylcellulose and 1.0 kg. of sodium, calcium poly(methyl vinyl ether/maleic acid). The blended premix was added to a heavy duty sigma blade-type mixer followed by the addition of 2 kg. of polyisobutylene. After mixing for ten minutes, an additional 2 kg. of polyisobutylene was added and mixing continued until a homogeneous dough was formed (about 10 to twenty minutes).

This dough mass while hot and soft was extruded and flattened. A sheet of polyethylene 1.5 mils (0.0308 mm) thickness was pressed over one side and silicone coated release paper on the other. The resultant mat was cut into the desired shape.

4

# 0 122 344

Examples 2—17

Following the procedure of Example 1 but employing the following ingredients on a weight percent basis other adhesive compositions within the scope of the invention are prepared.

| Ingredient | Examples | | | |
|---|---|---|---|---|
| | 2 | 3 | 4 | 5 |
| Polyisobutylene (Vistanex LM-MH) | 40 | 40 | 40 | 40 |
| Guar gum | 25 | — | — | 30 |
| Locust bean gum | — | — | — | — |
| Pectin | — | — | — | — |
| Karaya | — | — | — | — |
| Gelatin | — | — | — | — |
| Sodium carboxymethylcellulose | 10 | — | 30 | — |
| Cross-linked sodium carboxymethylcellulose (Aqualon R) | 15 | — | — | — |
| Starch-acrylonitrile graft copolymer (Grain Processing Corp. Polymer 35-A-100) | — | — | — | — |
| Cross-linked dextran (Sephadex CM-C50) | — | — | — | — |
| Poly(methyl vinyl ether/maleic acid), or its anhydride, or mixed calcium, sodium salt [Gantrez S-97, AN-169, or AT-955] | 10 (S-97) | 60 (AT-955) | 30 (AT-955) | 30 (AT-955) |
| Gluten | — | — | — | — |

| Ingredient | Examples | | | |
|---|---|---|---|---|
| | 6 | 7 | 8 | 9 |
| Polyisobutylene (Vistanex LM-MH) | 40 | 45 | 50 | 60 |
| Guar gum | — | — | 30 | 25 |
| Locust bean gum | — | 25 | — | — |
| Pectin | — | 15 | — | — |
| Karaya | — | — | — | — |
| Gelatin | — | — | — | — |
| Sodium carboxymethylcellulose | 17.2 | — | — | — |
| Cross-linked sodium carboxymethylcellulose (Aqualon R) | 25.6 | — | — | — |
| Starch-acrylonitrile graft copolymer (Grain Processing Corp. Polymer 35-A-100) | — | — | — | — |
| Cross-linked dextran (Sephadex CM-C50) | — | — | — | — |
| Poly(methyl vinyl ether/maleic acid), or its anhydride, or mixed calcium, sodium salt [Gantrez S-97, AN-169, or AT-955] | 17.2 (AT-955) | 15 (AT-955) | 20 (AN-169) | 15 (S-97) |
| Gluten | — | — | — | — |

5

| Ingredient | Examples | | | |
|---|---|---|---|---|
| | 10 | 11 | 12 | 13 |
| Polyisobutylene (Vistanex LM-MH) | 40 | 40 | 40 | 40 |
| Guar gum | — | 25 | 30 | 30 |
| Locust bean gum | — | — | — | — |
| Pectin | 15 | — | — | — |
| Karaya | — | 15 | — | — |
| Gelatin | 15 | — | — | — |
| Sodium carboxymethylcellulose | 15 | — | — | — |
| Cross-linked sodium carboxymethylcellulose (Aqualon R) | — | 10 | — | — |
| Starch-acrylonitrile graft copolymer (Grain Processing Corp. Polymer 35-A-100) | — | — | 10 | — |
| Cross-linked dextran (Sephadex CM-C50) | — | — | — | 10 |
| Poly(methyl vinyl ether/maleic acid), or its anhydride, or mixed calcium, sodium salt [Gantrez S-97, AN-169, or AT-955] | 15 (AT-955) | 10 (AT-955) | 20 (AT-955) | 20 (AT-955) |
| Gluten | — | — | — | — |

| Ingredient | Examples | | | |
|---|---|---|---|---|
| | 14 | 15 | 16 | 17 |
| Polyisobutylene (Vistanex LM-MH) | 35 | 40 | 50 | 40 |
| Guar gum | 25 | 25 | 20 | 20 |
| Locust bean gum | — | — | — | — |
| Pectin | 15 | — | — | — |
| Karaya | — | — | — | — |
| Gelatin | — | — | — | — |
| Sodium carboxymethylcellulose | — | 10 | 10 | — |
| Cross-linked sodium carboxymethylcellulose (Aqualon R) | 7.5 | 10 | 10 | 20 |
| Starch-acrylonitrile graft copolymer (Grain Processing Corp. Polymer 35-A-100) | 7.5 | — | — | — |
| Cross-linked dextran (Sephadex CM-C50) | — | — | — | — |
| Poly(methyl vinyl ether/maleic acid), or its anhydride, or mixed calcium, sodium salt [Gantrez S-97, AN-169, or AT-955] | 10 (AT-955) | — | — | — |
| Gluten | — | 15 | 10 | 20 |

# 0 122 344

Example 18
This example is directed to preparing an adhesive mass having the following composition:

| Ingredient | Percent by weight |
|---|---|
| Polyisobutylene (Vistanex LM-MH) | 32 |
| Guar Gum | 25 |
| Sodium carboxymethylcellulose | 12 |
| Cross-linked sodium carboxymethylcellulose (Aqualon R) | 13 |
| Sodium, calcium poly(methyl vinyl ether/maleic acid) [Gantrez AT-955] | 10 |
| Styrene-isoprene copolymer (Kraton 1107) | 8 |

A powder premix is prepared by blending 2.5 kg. of finely divided guar gum, 1.2 kg. of sodium carboxymethylcellulose, 1.3 kg. of cross-linked sodium carboxymethylcellulose, and 1.0 kg. of sodium, calcium poly(methyl vinyl ether/maleic acid).

The Kraton 1107 (0.8 kg.) is masticated in a high shear sigma mixer at 120°C until soft. To this is added 3.2 kg. of polyisobutylene and mixing is continued until the blend is homogeneous. The mass is cold to 30°C at which time the powdered premix is added. Mixing is continued until a homogeneous dough is formed.

This dough mass while hot and soft is extruded and flattened. A sheet of polyethylene 1.5 mils (0.0308 mm) thickness is pressed over one side and silicone coated release paper on the other. The resultant mat is cut into the desired shape.

Examples 19—25
Following the procedure of Example 18 but employing the following ingredients on a weight percent basis other adhesive compositions within the scope of the invention are prepared.

7

| Ingredient | Examples | | | | | | |
|---|---|---|---|---|---|---|---|
| | 19 | 20 | 21 | 22 | 23 | 24 | 25 |
| Polyisobutylene (Vistanex LM-MH) | 32 | — | 32 | 32 | — | 32 | — |
| Polyisobutylene (Vistanex LM-MS) | — | 36 | — | — | 36 | — | 36 |
| Guar gum | 25 | 15 | 30 | 25 | — | — | 25 |
| Locust Bean gum | — | — | — | — | — | 20 | — |
| Pectin | — | 15 | — | — | 15 | — | 10 |
| Karaya | — | — | — | — | — | 20 | — |
| Gelatin | — | — | — | — | 15 | — | — |
| Sodium carboxymethylcellulose | 13 | — | — | 12 | 15 | — | — |
| Cross-linked sodium carboxymethylcellulose (Aqualon R) | 12 | 10 | 18 | 13 | — | — | — |
| Starch-acrylonitrile graft copolymer (Grain Processing Corp. Polymer 35-A-100) | — | — | — | — | — | 10 | — |
| Cross-linked dextran (Sephadex CM-C50) | — | — | — | — | — | — | 10 |
| Poly(methylvinyl ether/maleic acid), or its anhydride, or mixed calcium, sodium salt [Gantrez S-97, AN-169, or AT-955] | 10 AT-955 | 15 AT-955 | 12 AT-955 | — | 10 S-97 | 10 AN-169 | 10 AT-955 |
| Gluten | — | — | — | 10 | — | — | — |
| Kraton 1107 | 8 | 9 | 8 | — | — | 8 | 9 |
| Butyl rubber (077) | — | — | — | 8 | — | — | — |
| Polyisobutylene (Vistanex L-100) | — | — | — | — | 9 | — | — |

**Claims**

1. An adhesive composition suitable for medical purposes comprising a substantially homogeneous mixture on a percent weight basis of from 30% to 70% of a pressure-sensitive viscous adhesive material and, optional by, a thermoplastic elastomer, said pressure-sensitive adhesive material being selected from natural rubber, silicone rubber, acrylonitrile rubber, polyurethane rubber, and polyisobutylenes and said optional thermoplastic elastomer being selected from medium molecular weight polyisobutylene, butyl rubber, and styrene copolymers; and from 3% to 60% by weight of one or more natural or synthetic polymers capable of developing elastomeric properties when hydrated and selected from gluten and long chain polymers of methyl vinyl ether/maleic acid.

2. The adhesive composition of Claim 1 wherein said long chain polymer is selected from poly(methyl vinyl ether/maleic acid), poly(methyl vinyl ether/maleic anhydride), and calcium, sodium poly(methyl vinyl ether/maleic acid).

3. The adhesive composition of Claim 2 wherein said pressure sensitive viscous adhesive material is low molecular weight polyisobutylene and said polyisobutylene is present at from 40% to 50% by weight of said composition.

4. The adhesive composition of Claim 3 consisting essentially of, on a weight percent basis, about 40% polyisobutylene and about 60% calcium, sodium poly(methyl vinyl ether/maleic acid).

5. An adhesive composition suitable for medical purposes comprising a substantially homogeneous mixture on a percent weight basis of from 30% to 70% of a pressure-sensitive viscous adhesive material and, optionally, a thermoplastic elastomer, said pressure sensitive viscous adhesive material being selected from natural rubber, silicone rubber, acrylonitrile rubber, polyurethane rubber, and polyisobutylenes and said optional thermoplastic elastomer being selected from medium molecular weight polyisobutylenes, butyl rubber, and styrene copolymers; from 3% to 60% by weight of one or more natural or synthetic polymers capable of developing elastomeric properties when hydrated and selected from gluten and long chain polymers of methyl vinyl ether/maleic acid; up to 50% by weight of one or more water soluble hydrocolloids selected from sodium carboxymethylcellulose, pectin, gelatin, guar gum, locust bean gum, gum karaya, and mixtures thereof; and up to 30% by weight of one or more water swellable cohesive strengthening agents selected from water insoluble cross-linked sodium carboxymethylcellulose, water insoluble starch-acrylonitrile graft polymer, and water insoluble cross-linked dextran, provided that the water soluble hydrocolloids and water insoluble cohesive strengthening agents together are no more than 60% by weight of said adhesive composition.

6. The adhesive composition of Claim 5 wherein said long chain polymer is selected from poly(methyl vinyl ether/maleic acid), poly(methyl vinyl ether/maleic anhydride), and calcium, sodium poly(methyl vinyl ether/maleic acid).

7. The adhesive composition of Claim 6 wherein said pressure sensitive viscous adhesive material is low molecular weight polyisobutylene.

8. An adhesive composition suitable for medical purposes comprising a substantially homogeneous mixture on a percent weight basis of from 40% to 50% of a pressure-sensitive adhesive component comprising low molecular weight polyisobutylene and optionally, a thermoplastic elastomer selected from medium molecular weight polyisobutylene, butyl rubber, and styrene-isoprene-styrene copolymers; from 10% to 35% of a polymer selected from poly(methyl vinyl ether/maleic acid), poly(methyl vinyl ether/maleic anhydride), and calcium, sodium poly(methyl vinyl ether/maleic acid); from 15% to 40% of one or more water soluble hydrocolloids selected from sodium carboxymethylcellulose, pectin, gelatin, guar gum, locust bean gum, gum karaya, and mixtures thereof; and up to 30% of one or more water swellable cohesive strengthening agents selected from water insoluble cross-linked sodium carboxymethylcellulose, water insoluble starch-acrylonitrile graft copolymer, water insoluble cross-linked dextran, and mixtures thereof, provided that the water soluble hydrocolloids and water swellable cohesive strengthening agents together are no more than 55% by weight of said composition.

9. The adhesive composition of Claim 8 wherein said water soluble hydrocolloids are selected from guar gum, sodium carboxymethylcellulose, and mixtures thereof, and said cohesive strengthening agent, if present, is cross-linked sodium carboxymethylcellulose.

10. The adhesive composition of Claim 9 consisting essentially of, on a percent weight basis, about 40% low molecular weight polyisobutylene, about 25% guar gum, about 10% sodium carboxymethylcellulose, about 15% cross-linked sodium carboxymethylcellulose, and about 10% calcium, sodium poly(methyl vinyl ether/maleic acid).

11. The adhesive composition of Claim 9 consisting essentially of, on a weight percent basis, about 40% low molecular weight polyisobutylene, about 25% guar gum, about 10% sodium carboxymethylcellulose, about 15% cross-linked sodium carboxymethylcellulose, and about 10% poly(methyl vinyl ether/maleic acid).

12. The adhesive composition of Claim 9 consisting essentially of, on a percent weight basis, about 40% low molecular weight polyisobutylene, about 30% sodium carboxymethylcellulose, and about 30% calcium, sodium poly(methyl vinyl ether/maleic acid).

13. The adhesive composition of Claim 9 consisting essentially of, on a weight percent basis, about 40% low molecular weight polyisobutylene, about 30% guar gum, and about 30% calcium, sodium poly(methyl vinyl ether/maleic acid).

14. The adhesive composition of Claim 9 consisting essentially of, on a weight percent basis, about 40% low molecular weight polyisobutylene, about 17.2% sodium carboxymethylcellulose, about 25.6% cross-linked sodium carboxymethylcellulose, and about 17.2% calcium, sodium poly(methyl vinyl ether/maleic acid).

15. A skin barrier comprising an adhesive layer having a thin continuous or discontinuous polymeric film laminated to one surface of the adhesive, said adhesive being in accordance with any preceding claim.

16. A skin barrier of Claim 15 wherein said polymeric film is polyethylene.


**Patentansprüche**

1. Klebemasse für medizinische Zwecke, enthaltend ein im wesentlichen homogenes Gemisch aus 30 bis 70 Gewichtsprozent eines viskosen Haftklebers und gegebenenfalls einem thermoplastischen Elastomer, wobei der Haftkleber natürlicher Kautschuk, Siliconkautschuk, Acrylnitrilkautschuk, Polyurethankautschuk oder Polyisobutylen ist und das gegebenenfalls vorliegende thermoplastische Elastomer Polyisobutylen von mittlerem Molekulargewicht, Butylkautschuk oder ein Styrol-Copolymeres ist; und 3 bis 60 Gewichtsprozent eines oder mehrerer natürlicher oder synthetischer Polymere, die, wenn sie hydratisiert werden, elastomere Eigenschaften entwickeln, und die aus Gluten oder langkettigen Polymeren aus Methylvinyläther/Maleinsäure bestehen.

2. Klebemasse nach Anspruch 1, in der das langkettige Polymer Poly(Methylvinyläther/Maleinsäure), Poly(Methylvinyläther/Maleinsäureanhydrid) oder Calcium-Natrium-Poly-(Methylvinyläther/Maleinsäure) ist.

3. Klebemasse nach Anspruch 2, in der der viskose Haftkleber ein niedermolekulares Polyisobutylen ist und das Polyisobutylen in einer Menge von 40 bis 50 Gewichtsprozent der Masse vorliegt.

4. Klebemasse nach Anspruch 3, im wesentlichen bestehend aus etwa 40 Gewichtsprozent Polyisobutylen und etwa 60 Gewichtsprozent Calcium-Natrium-Poly(Methylvinyläther/Maleinsäure).

**0 122 344**

5. Klebemasse für medizinische Zwecke, enthaltend ein im wesentlichen homogenes Gemisch aus 30 bis 70 Gewichtsprozent eines viskosen Haftklebers und gegebenenfalls einem thermoplastischen Elastomer, wobei der viskose Haftkleber natürlicher Kautschuk, Siliconkautschuk, Acrylnitrilkautschuk, Polyurethankautschuk oder Polyisobutylen ist und das gegebenenfalls vorliegende thermoplastische Elastomer Polyisobutylen von mittlerem Molekulargewicht, Butylkautschuk oder ein Styrol-Copolymeres ist; 3 bis 60 Gewichtsprozent eines oder mehrerer natürlicher oder synthetischer Polymere, die, wenn sie hydratisiert werden, elastomere Eigenschaften entwickeln, und die aus Gluten oder langkettigen Polymeren von Methylvinyläther/Maleinsäure bestehen; bis zu 50 Gewichtsprozent eines oder mehrerer wasserlöslicher Hydrokolloide, nämlich Natriumcarboxymethylcellulose, Pektin, Gelatine, Guarmehl, Karobegummi, Karayagummi oder Gemische davon; und bis zu 30 Gewichtsprozent eines oder mehrerer in Wasser quellbarer kohäsiver Verstärkungsmittel, nämlich in Wasser unlösliche quervernetzte Natriumcarboxymethylcellulose, in Wasser unlösliches Stärke-Acrylnitrilpfropf-Copolymer oder in Wasser unlösliches quervernetztes Dextran, mit der Maßgabe, daß die wasserlöslichen Hydrokolloide und die in Wasser unlöslichen kohäsiven Verstärkungsmittel zusammen nicht mehr als 60 Gewichtsprozent der Klebemasse ausmachen.

6. Klebemasse nach Anspruch 5, in der das langkettige Polymer Poly(Methylvinyläther/Maleinsäure), Poly(Methylvinyläther/Maleinsäureanhydrid) oder Calcium-Natrium-Poly(Methylvinyläther/Maleinsäure) ist.

7. Klebemasse nach Anspruch 6, in der der viskose Haftkleber niedermolekulares Polyisobutylen ist.

8. Klebemasse für medizinische Zwecke, enthaltend ein im wesentlichen homogenes Gemisch aus 40 bis 50 Gewichtsprozent einer Haftkleber-Komponente, die niedermolekulares Polyisobutylen und gegebenenfalls ein thermoplastisches Elastomer, nämlich ein Polyisobutylen von mittlerem Molekulargewicht, Butylkautschuk oder Styrol-Isopren-Styrol-Copolymere enthält; 10 bis 35 Gewichtsprozent eines Polymers, nämlich Poly(Methylvinyläther/Maleinsäure), Poly(Methylvinyläther/Maleinsäureanhydrid) oder Calcium-Natrium-Poly(Vinylmethyläther/Maleinsäure); 15 bis 40 Gewichtsprozent eines oder mehrerer wasserlöslicher Hydrokolloide, nämlich Natriumcarboxymethylcellulose, Pektin, Gelatine, Guarmehl, Karobegummi, Karayagummi oder Gemische davon; und bis zu 30 Gewichtsprozent eines oder mehrerer in Wasser quellbarer kohäsiver Verstärkungsmittel, nämlich in Wasser unlösliche quervernetzte Natriumcarboxymethylcellulose, in Wasser unlösliches Stärke-Acrylnitrilpfropf-Copolymer, in Wasser unlösliches quervernetztes Dextran oder Gemische davon, mit der Maßgabe, daß die wasserlöslichen Hydrokolloide und die in Wasser quellbaren kohäsiven Verstärkungsmittel zusammen nicht mehr als 55 Gewichtsprozent der Klebemasse ausmachen.

9. Klebemasse nach Anspruch 8, in der die wasserlöslichen Hydrokolloide Guarmehl, Natriumcarboxymethylcellulose oder Gemische davon sind, und das kohäsive Verstärkungsmittel, falls vorhanden, quervernetzte Natriumcarboxymethylcellulose ist.

10. Klebemasse nach Anspruch 9, im wesentlichen bestehend aus etwa 40 Gewichtsprozent niedermolekularem Polyisobutylen, etwa 25 Gewichtsprozent Guarmehl, etwa 10 Gewichtsprozent Natriumcarboxymethylcellullose, etwa 15 Gewichtsprozent quervernetzter Natriumcarboxymethylcellulose und etwa 10 Gewichtsprozent Calcium-Natrium-Poly-(Methylvinyläther/Maleinsäure).

11. Klebemasse nach Anspruch 9, im wesentlichen bestehend aus etwa 40 Gewichtsprozent niedermolekularem Polyisobutylen, etwa 25 Gewichtsprozent Guarmehl, etwa 10 Gewichtsprozent Natriumcarboxymethylcellulose, etwa 15 Gewichtsprozent quervernetzter Natriumcarboxymethylcellulose und etwa 10 Gewichtsprozent Poly(Methylvinyläther/Maleinsäure).

12. Klebemasse nach Anspruch 9, im wesentlichen bestehend aus etwa 40 Gewichtsprozent niedermolekularem Polyisobutylen, etwa 30 Gewichtsprozent Natriumcarboxymethylcellulose und etwa 30 Gewichtsprozent Calcium-Natrium-Poly(Methylvinyläther/Maleinsäure).

13. Klebemasse nach Anspruch 9, im wesentlichen bestehend aus etwa 40 Gewichtsprozent niedermolekularem Polyisobutylen, etwa 30 Gewichtsprozent Guarmehl und etwa 30 Gewichtsprozent Calcium-Natrium-Poly(Methylvinyläther/Maleinsäure).

14. Klebemasse nach Anspruch 9, im wesentlichen bestehend aus etwa 40 Gewichtsprozent niedermolekularem Polyisobutylen, etwa 17.2 Gewichtsprozent Natriumcarboxymethylcellulose, etwa 25,6 Gewichtsprozent quervernetzter Natriumcarboxymethylcellulose und etwa 17,2 Gewichtsprozent Calcium-Natrium-Poly(Methylvinyläther/Maleinsäure).

15. Hautschranke, enthaltend eine Klebeschicht mit einem dünnen kontinuierlichen oder diskontinuierlichen Polymerfilm, der auf eine Oberfläche der Klebeschicht aufgebracht ist, wobei die Klebeschicht einem der vorangehenden Ansprüche entspricht.

16. Hautschranke nach Anspruch 15, in der der Polymerfilm aus Polyäthylen besteht.

**Revendications**

1. Composition adhésive utilisable à des fins médicales, comprenant un mélange sensiblement homogène constitué, en pourcentage en poids, de 30% à 70% d'une matière adhésive visqueuse sensible à la pression et, facultativement, d'un élastomère thermoplastique, ladite matière adhésive sensible à la pression étant choisie entre le caoutchouc naturel, le caoutchouc de silicone, le caoutchouc d'acrylonitrile, le caoutchouc de polyuréthane, et les polyisobutylènes, et ledit élastomère thermoplastique facultatif étant choisi entre le polyisobutylène de poids moléculaire intermédiaire, le caoutchouc butyle, et les copolymères du styrène; et de 3% à 60% en poids d'un ou de plusieurs polymères naturels ou synthétiques capables de développer des propriétés élastomères quand ils sont hydratés, et choisis entre le gluten et les polymères à longue chaîne de méthyl vinyl éther et d'acide maléique.

2. Composition adhésive selon la revendication 1, dans laquelle ledit polymère à longue chaîne est choisi entre les polymères de méthyl vinyl éther et d'acide maléique, les polymères de méthyl vinyl éther et d'anhydride maléique, et les sels de calcium et de sodium de polymères de méthyl vinyl éther et d'acide maléique.

3. Composition adhésive selon la revendication 2, dans laquelle ladite matière adhésive visqueuse sensible à la pression est un polyisobutylène de bas poids moléculaire et ledit polyisobutylène représente de 40% à 50% du poids de ladite composition.

4. Composition adhésive selon la revendication 3, se composant essentiellement, en pourcentage en poids, de 40% environ de polyisobutylène et de 60% environ de sel de calcium et de sodium de polymère de méthyl vinyl éther et l'acide maléique.

5. Composition adhésive utilisable à des fins médicales, comprenant un mélange sensiblement homogène constitué, en pourcentage en poids, de 30% à 70% d'une matière adhésive visqueuse sensible à la pression et, facultativement, d'un élastomère thermoplastique, ladite matière adhésive visqueuse sensible à la pression étant choisie entre le caoutchouc naturel, le caoutchouc de silicone, le caoutchouc d'acrylonitrile, le caoutchouc de polyuréthane, et les polyisobutylènes, et ledit élastomère thermoplastique facultatif étant choisi entre les polyisobutylènes de poids moléculaire intermédiaire, le caoutchouc butyle, et les copolymères du styrène; de 3% à 60% en poids d'un ou de plusieurs polymères naturels ou synthétiques capables de développer des propriétés élastomères quand ils sont hydratés, et choisis entre le gluten et les polymères à longue chaîne de méthyl vinyl éther et d'acide maléique; jusqu'à 50% en poids d'un ou de plusieurs hydrocolloïdes solubles dans l'eau choisis entre la carboxyméthylcellulose sodique, la pectine, la gélatine, le guar, la gomme tragasol, la gomme karaya, et leurs mélanges, et jusqu'à 30% en poids d'un ou de plusieurs agents de renforcement de la cohésion, gonflant dans l'eau, qui sont choisis entre la carboxyméthylcellulose sodique réticulée insoluble dans l'eau, un copolymère greffé d'amidon et d'acrylonitrile, insoluble dans l'eau, et le dextranne réticulé insoluble dans l'eau, à condition que les hydrocolloïdes solubles dans l'eau et les agents de renforcement de la cohésion insolubles dans l'eau ne représentent ensemble pas plus de 60% du poids de ladite composition adhésive.

6. Composition adhésive selon la revendication 5, dans laquelle ledit polymère à longue chaîne est choisi entre les polymères de méthyl vinyl éther et d'acide maléique, les polymères de méthyl vinyl éther et d'anhydride maléique, et les sels de calcium et de sodium de polymères de méthyl vinyl éther et d'acide maléique.

7. Composition adhésive selon la revendication 6, dans laquelle ladite matière adhésive visqueuse sensible à la pression est un polyisobutylène de bas poids moléculaire.

8. Composition adhésive utilisable à des fins médicales, comprenant un mélange sensiblement homogène constitué, en pourcentage en poids, de 40% à 50% d'un constituant adhésif sensible à la pression comprenant un polyisobutylène de bas poids moléculaire et, facultativement, un élastomère thermoplastique choisi entre un polyisobutylène de poids moléculaire intermédiaire, le caoutchouc butyle, et les copolymères de styrène-isoprène-styrène; de 10% à 35% d'un polymère choisi entre les polymères de méthyl vinyl éther et d'acide maléique, les polymères de méthyl vinyl éther et d'anhydride maléique, et les sels de calcium et de sodium de polymères de méthyl vinyl éther et d'acide maléique; de 15% à 40% d'un ou de plusieurs hydrocolloïdes solubles dans l'eau choisis entre la carboxyméthylcellulose sodique, la pectine, la gélatine, le guar, la gomme tragasol, la gomme karaya, et leurs mélanges; et jusqu'à 30% d'un ou de plusieurs agents de renforcement de la cohésion, gonflant dans l'eau, qui sont choisis entre la carboxyméthylcellulose sodique réticulée insoluble dans l'eau, un copolymère greffé d'amidon et d'acrylonitrile insoluble dans l'eau, le dextranne réticulé insoluble dans l'eau, et leurs mélanges, à condition que les hydrocolloïdes solubles dans l'eau et les agents de renforcement de la cohésion gonflant dans l'eau ne représentent ensemble pas plus de 55% du poids de ladite composition.

9. Composition adhésive selon la revendication 8, dans laquelle lesdits hydrocolloïdes solubles dans l'eau sont choisis entre le guar, la carboxyméthylcellulose sodique, et leurs mélanges, et ledit agent de renforcement de la cohésion, s'il est présent, est la carboxyméthylcellulose sodique réticulée.

10. Composition adhésive selon la revendication 9, se composant essentiellement, en pourcentage en poids, d'environ 40% de polyisobutylène de bas poids moléculaire, d'environ 25% de guar, d'environ 10% de carboxyméthylcellulose sodique, d'environ 15% de carboxyméthylcellulose sodique réticulée, et d'environ 10% de sel de calcium et de sodium de polymère de méthyl vinyl éther et d'acide maléique.

11. Composition adhésive selon la revendication 9, se composant essentiellement, en pourcentage en poids, d'environ 40% de polyisobutylène de bas poids moléculaire, d'environ 25% de guar, d'environ 10% de carboxyméthylcellulose sodique, d'environ 15% de carboxyméthylcellulose sodique réticulée, et d'environ 10% de polymère de méthyl vinyl éther et d'acide maléique.

12. Composition adhésive selon la revendication 9, se composant essentiellement, en pourcentage en poids, d'environ 40% de polyisobutylène de bas poids moléculaire, d'environ 30% de carboxyméthylcellulose sodique, et d'environ 30% de sel de calcium et de sodium de polymère de méthyl vinyl éther et d'acide maléique.

13. Composition adhésive selon la revendication 9, se composant essentiellement, en pourcentage en poids, d'environ 40% de polyisobutylène de bas poids moléculaire, d'environ 30% du guar, et d'environ 30% de sel de calcium et de sodium de polymère de méthyl vinyl éther et d'acide maléique.

14. Composition adhésive selon la revendication 9, se composant essentiellement, en pourcentage en poids, d'environ 40% de polyisobutylène de bas poids moléculaire, d'environ 17,2% de carboxyméthylcellulose sodique, d'environ 25,6% de carboxyméthylcellulose sodique réticulée, et d'environ 17,2% de sel de calcium et de sodium de polymère de méthyl vinyl éther et d'acide maléique.

15. Barrière dermique comprenant une couche adhésive comportant une fine pellicule polymère continue ou discontinue plaquée sur l'une des surfaces de l'adhésif, ledit adhésif étant conforme à l'une quelconque des revendications précédentes.

16. Barrière dermique selon la revendication 15, dans laquelle ladite pellicule polymère est une pellicule de polyéthylène.

FIG. 1

FIG. 2